# EUROPÄISCHE PATENTSCHRIFT

(11) **EP 0 751 754 B1**
(45) Veröffentlichungstag und Bekanntmachung des Hinweises auf die Patenterteilung: **20.08.1997**
(21) Anmeldenummer: 95920810.9
(22) Anmeldetag: 15.05.1995
(51) Int. Cl.: A61F 5/01

(54) **KNIEORTHESE**
KNEE ORTHESIS
ORTHESE POUR LE GENOU

(30) Priorität: 30.05.1994 DE 4418855
(43) Veröffentlichungstag der Anmeldung: 08.01.1997
(73) Patentinhaber: FERD. HAUBER GmbH & CO. KG, D-72622 Nürtingen (DE)
(72) Erfinder: AHLBÄUMER, Georg, D-52080 Aachen (DE); LOMMER, Willibald, D-80339 München (DE); BERKOWITSCH, Ewald, D-73230 Kirchheim/Teck (DE)
(74) Vertreter: Becker, Maria, Dipl.-Phys.
(86) Internationale Anmeldenummer: EP9501833
(87) Internationale Veröffentlichungsnummer: WO9532692

(56) Entgegenhaltungen:
- EP-A- 0 297 766
- EP-A- 0 413 523
- US-A- 4 854 308
- US-A- 5 135 471

## Beschreibung

Die Erfindung betrifft eine Knieorthese mit den Merkmalen des Oberbegriffs von Anspruch 1.

Knieorthesen dieser Art dienen zur Behandlung einer Vielzahl möglicher Knieverletzungen. Die beiden halbschalenförmigen, an den Ober- bzw. Unterschenkel anlegbaren und über seitliche Gelenke in einer Ebene miteinander schwenkbar verbundenen Orthesenteile bestehen hierbei aus Kunststoffteilen, die jeweils mittels Spannbändern am Bein festlegbar sind.

Um einen möglichst zufriedenstellenden Beinsitz von Knieorthesen zu erzielen, werden dabei Orthesenteile auf Lager gehalten, die in ihrer Krümmung unterschiedliche Beinumfangsmaße aufweisen. Nach Verordnen einer Knieorthese läßt sich diese dann durch Auswahl geeigneter Orthesenteile dem Bein eines zu behandelnden Knies einigermaßen anpassen. Allerdings läßt sich auch bei einer sehr sorgfältigen Anpassung von Orthesenteilen in den meisten Fällen der angestrebte optimale Sitz einer Knieorthese über den Verlauf eines Tages nicht aufrecht erhalten. Dies liegt darin begründet, daß sich der Beinumfang tagsüber verändert. Besonders gravierende Beinumfangsänderungen werden dabei durch Schwellungen nach Knieeingriffen verursacht. Das Ausmaß solcher Schwellungen der Beinmuskulatur kann sich dabei während eines Tages beträchtlich ändern.

Eine derartige Knieorthese ist aus der EP-A 297 766, die als Basis für den Oberbegriff des Anspruchs 1 verwendet ist, bekannt. Diese zeigt halbschalenförmige Orthesenteile, die unmittelbar an Ober- bzw. Unterschenkel anzulegen sind und die Ober Verbindungselemente schwenkbar verbunden sind, wobei die Verbindungselemente an unterschiedliche Kniegelenke anpaßbar sind.

Es ist deshalb Aufgabe der Erfindung, eine Knieorthese der im Oberbegriff des Anspruches 1 erläuterten Art anzugeben, die sich an gegebene bzw. durch Muskelschwellungen bedingte Beinumfangsänderungen leicht anpassen läßt.

Diese Aufgabe wird erfindungsgemäß durch die kennzeichnenden Merkmale des Anspruches 1 gelöst.

Die erfindungsgemäße Konstruktion ermöglicht somit auf einfache Weise eine Weiteneinstellung der Orthesenteile quer zur Beinlängsrichtung und damit eine individuelle, anatomisch richtige Anpassung an sich einstellende Beinschwellungen, wobei sich die Position der die beiden Orthesenteile miteinander verbindenden Gelenke in Beinlängsrichtung nicht ändert, so daß bei derartigen Weitenveränderungen der Orthesenteile die Isometrie der Gelenksbewegung voll erhalten bleibt.

Eine Ausgestaltung der Erfindung nach Anspruch 2 bietet hierbei den besonderen Vorteil, die Orthese an Beinen mit Varus- und Valgusdeformitäten leicht anpassen zu können, so daß sie für alle denkbaren Beinstellungen individuell und problemlos passend ist.

Ausgestaltungen der Erfindung gemäß den Ansprüchen 3 bis 5 ermöglichen auf technisch besonders einfache Weise notwendige Weitenkorrekturen beider Orthesenteile.

Eine weitere, vorteilhafte Ausgestaltung der Erfindung ist Gegenstand des Anspruches 6. Derart konzipierte Orthesenteile ermöglichen deren Festlegung auf längeren Beinabschnitten, wobei es dann, gemäß Anspruch 7, ausreichend ist, lediglich das dem Knie benachbarte Teilstück beider Orthesenteile in der Weite verstellbar auszubilden, da Schwellungen der Beinmuskulatur in der Hauptsache im unmittelbaren Bereich verletzter Knie auftreten.

Vorteilhafte gegenseitige Verbindungen beider Teilstücke jedes Orthesenteils sind Gegenstand der Ansprüche 8 und 9.

In weiterer Ausgestaltung der Erfindung ist eine Befestigung der Spannbänder beider Orthesenteile gemäß Anspruch 10 vorgesehen.

Eine solche schwenkbare Anordnung von die Spannbänder haltenden Halteösen an den Orthesenteilen ermöglicht beim Anlegen der vorteilhaft mit Klettverschlüssen ausgestatteten Spannbänder eine der Beinmuskulatur individuell anpassbare Winkeleinstellung für optimalen Bandsitz, was wiederum während eines ganzen Tages einen einwandfreien Sitz der Knieorthese am Bein gewährleistet.

Schließlich ist es günstig, wenn, gemäß Anspruch 11, beide Orthesenteile gegen die Wirkung einer Rückstellkraft aus der Orthesenstrecklage verschwenkbar sind, was sich auf einfache Weise gemäß Anspruch 12 verwirklichen läßt. Ein derartiger Kniestreckzug ist insbesondere angezeigt bei leichten Paresen oder zu Rehabilitationszwecken.

In der Zeichnung ist ein Ausführungsbeispiel einer erfindungsgemäßen Knieorthese dargestellt. Es zeigen:
- Figur 1: eine Vorderansicht der an ein Bein angelegten Knieorthese,
- Figur 2: eine Seitenansicht der Knieorthe se,
- Figur 3: eine Vorderansicht des oberen Orthesenteils zur Veranschaulichung seiner möglichen Weiten- und Längenveränderungen.

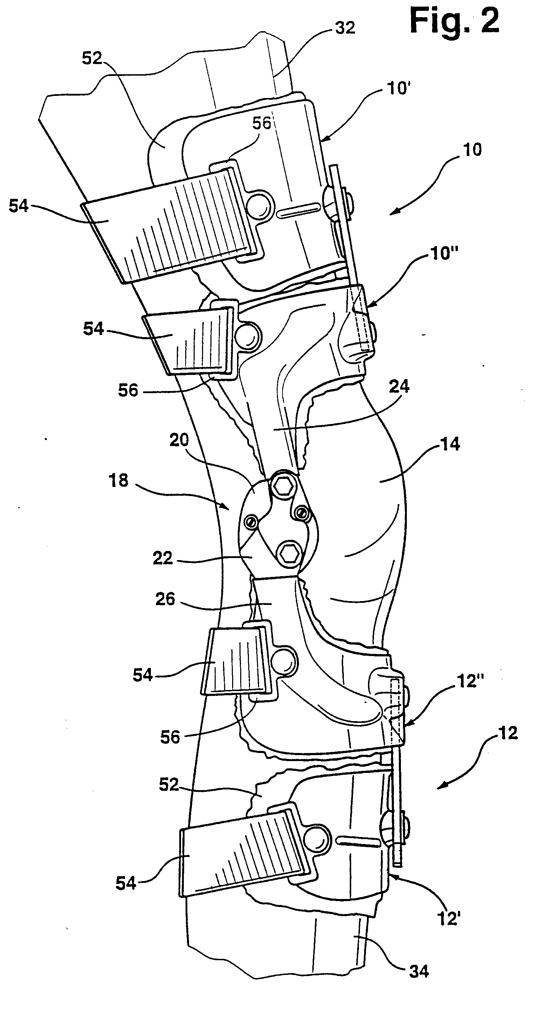

Die gezeigte Knieorthese weist zwei etwa halbschalenförmige, als Ganzes mit 10 und 12 bezeichnete Orthesenteile auf. Diese sind über zu beiden Seiten eines zu stabilisierenden Knies 14 vorgesehene Gelenke 16 und 18 in einer Ebene (Zeichenebene der Figur 2) miteinander schwenkbar verbunden.

Vorzugsweise handelt es sich um sogenannte polyzentrische Gelenke, welche in der Lage sind, die physiologische Rollgleitbewegung des Knies 14 zu imitieren. Solche Gelenke sind bekannt und Gegenstand der AT-PS 384 733 B.

Jede Gelenkhälfte 20 bzw. 22 ist am einen Ende einer Kniegelenkschiene 24 bzw. 26 angeformt, die ihrerseits am oberen bzw. unteren Orthesenteil 10 bzw. 12 gehalten ist. Beide Orthesenteile 10, 12 sind vorzugsweise jeweils durch zwei halbschalenförmige Teilstücke 10', 10'' bzw. 12', 12'' gebildet, die in Beinlängsrichtung im Abstand voneinander angeordnet und derart miteinander verbunden sind, daß deren Abstand in einem festgelegten Umfang stufenlos veränderbar ist.

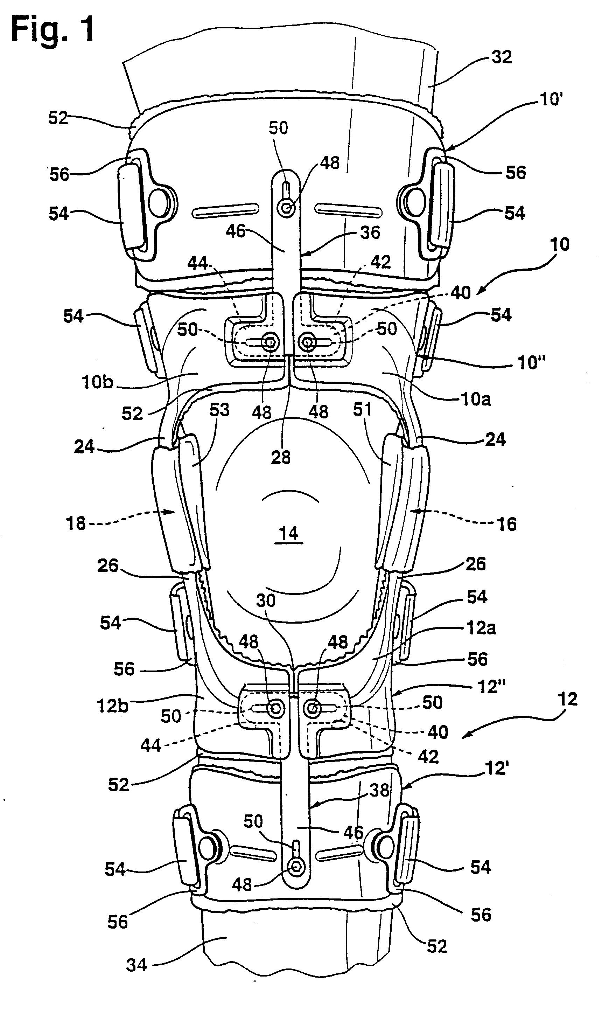

Wie Figur 1 zeigt, sind vorzugsweise lediglich die beiden, nächst dem Knie 14 liegenden Orthesenteilstücke 10'' und 12'' längs ihres Scheitels 28 bzw. 30 geteilt und quer zur Beinlängsrichtung voneinander unabhängig zueinander stufenlos verstellbar.

Die Aufteilung beider vorzugsweise durch Kunststofformteile gebildeten Orthesenteile 10, 12 in die Teilstücke 10', 10'' bzw. 12', 12'' und die Verstellbarkeit der Teilstücke 10' und 12' in Beinlängsrichtung erlaubt ein Anlegen der Knieorthese über einen entsprechend großen, individuell einstellbaren

Abschnitt am Beinober- bzw. -unterschenkel 32 bzw. 34. Die Teilung der Orthesenteilstücke 10'', 12'' entlang ihres Scheitels 28 bzw. 30 ermöglicht deren individuelle Anpassung an Schwellungen der Beinmuskulatur im Kniebereich.

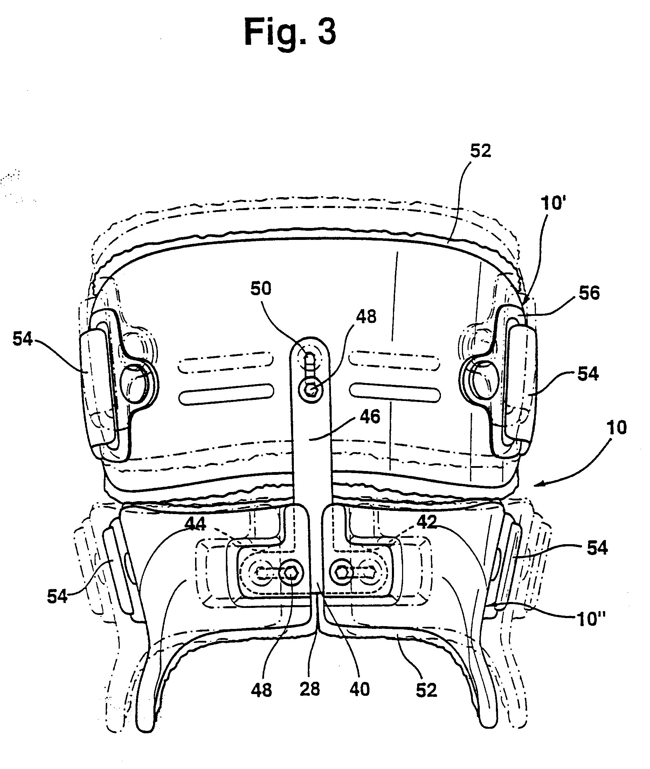

Wie aus Figur 3 ersichtlich ist, lassen sich damit die Orthesenteile 10, 12 insbesondere an Varus- und Valgusdeformitäten und sich tagsüber verändernde Umfänge von Ober- bzw. Unterschenkel 32 bzw. 34 respektive Muskelschwellungen optimal anpassen.

Die gegenseitige Verbindung der beiden Hälften 10a, 10b bzw. 12a, 12b der Orthesenteilstücke 10'' und 12'' und diejenige der beiden Orthesenteilstücke 10', 10'' und 12', 12'' zueinander ist vorzugsweise jeweils durch ein einziges Verbindungselement 36 bzw. 38 bewerkstelligt. Dasselbe ist hierzu T-förmig ausgebildet. Das den T-Balken 40 bildende Teilstück der Verbindungselemente 36, 38 greift vorzugsweise in jeweils eine taschenartige Ausnehmung 42 bzw. 44 der Hälften 10a, 10b bzw. 12a, 12b der Orthesenteilstücke 10'' und 12'' ein, während deren sich in Beinlängsrichtung erstreckender Steg 46 zur gegenseitigen Verbindung beider Orthesenteilstücke 10', 10'' bzw. 12', 12'' dient.

In die Hälften 10a, 10b bzw. 12a, 12b der innen liegenden Orthesenteilstücke 10'', 12'' sowie die einstückigen, außen liegenden Orthesenteilstücke 10' und 12' ist jeweils eine vorzugsweise ein Innenmehrkant aufweisende Spannschraube 48 eingeschraubt, die jeweils einen Längsschlitz 50 des betreffenden Verbindungselementes 36 bzw. 38 durchsetzt.

Zur Anpassung der Knieorthese an den Kniebereich lassen sich bei gelöster Spannschraube 48 die beiden Hälften 10a, 10b bzw. 12a, 12b der Orthesenteilstücke 10'', 12'' einzeln oder gemeinsam relativ zum T-Balken 40 des Verbindungselements 36 bzw. 38 und das Orthesenteilstück 10' bzw 12' relativ zu dessen Steg 46 entsprechend verschieben und durch Anziehen der Spannschrauben 48 am Verbindungselement 36 bzw. 38 festlegen.

Zur Erzielung eines angenehmen Tragekomforts sind die Orthesenteile 10 und 12 innenseitig mit einem vorzugsweise mit einer Frotteeauflage versehenen Schaumstoffpolster 52 und im Bereich der seitlichen Gelenke 16, 18 mit vorzugsweise abnehmbaren Polstern 51 und 53 ausgestattet. Außerdem sind die Orthesenteilstücke 10', 10'' sowie 12', 12'' zum Anlegen der Knieorthese an ein Bein jeweils mit zwei einander gegenüberliegend befestigten Spannbändern 54 versehen, die mittels Klettverschlüssen gegenseitig verbindbar sind.

Diese Spannbänder 54 sind an den Orthesenteilstücken 10', 10'' sowie 12', 12'' durch an diesen vorzugsweise schwenkbar befestigten Halteösen 56 gehalten. Diese Art der Ösenbefestigung bietet den Vorteil, daß sich die Spannbänder 54 durch formschlüssiges Anlegen an Beinumfangsabschnitte besonders günstig an die anatomische Form der Beinmuskulatur anpassen können und deshalb ein Verrutschen der Knieorthese über den gesamten Tag wirksam vermieden wird.

Durch den Einsatz von Kunststoff für die Orthesenteile 10 und 12 sowie Titan zur Herstellung der Verbindungselemente 36, 38 und der Gelenke 16, 18 einschließlich der in den Kunststoff der Orthesenteile 10, 12 eingebetteten Kniegelenkschienen 24, 26 konnte eine sich durch ein besonders geringes Gewicht auszeichnende und dementsprechende leicht zu handhabende, wartungsfreie Konstruktion geschaffen werden.

Die erläuterte Knieorthese ist zur konservativen Behandlung von Kreuzband- und Seitenbandverletzungen ebenso geeignet wie bei kombinierten Instabilitäten wie beispielsweise anteromediale Translations-Rotationsinstabilität, posterola terale Rotationsinstabilität, Hyperpressionssymptomatik bei Valgus-/Varusdeformität wie beispielsweise bei PCP, des weiteren beispielsweise zur Stabilisierung bei neurologischen Erkrankungen wie Multiple Sklerose, Muskeldystrophie, tiefe Paraplegie/Spastik, Hemiplegie und neurologisch bedingter Quadricepsatrophie, und schließlich zur postoperativen Behandlung von Kreuzbandrekonstruktionen, komplexen Knieverletzungen, Seitenbandreinserationen, bedingt bei gelenknahen Osteotomien bzw. Frakturen.

## Patentansprüche

1. Knieorthese, mit etwa zwei halbschalenförmigen, unmittelbar an den Ober- bzw. Unterschenkel(32 bzw. 34) anlegbaren und über seitliche Gelenke (16, 18) in einer Ebene miteinander verschwenkbar verbundenen Orthesenteilen (10, 12),
**dadurch gekennzeichnet, daß**
wenigstens Teilstücke (10'' bzw. 12'') der Orthesenteile (10, 12) längs ihres Scheitels (28 bzw. 30) geteilt und die beiden Orthesenteilhälften (10a, 10b 12a, 12b) quer zur Beinlängsrichtung relativ zueinander verstellbar und feststellbar sind.

2. Knieorthese nach Anspruch 1, dadurch gekennzeichnet, daS beide Hälften (10a, 10b bzw. 12a, 12b) der Orthesenteile (10, 12) voneinander unabhängig und zueinander stufenlos verstellbar sind.

3. Knieorthese nach Anspruch 1 oder 2, dadurch gekennzeichnet, daß beide Hälften (10a, 10b bzw. 12a, 12b) der Orthesenteile (10, 12) über eine sich in Beinquerrichtung erstreckende Verbindungsleiste (40) miteinander verbunden und an dieser verstellbar und feststellbar geführt sind.

4. Knieorthese nach Anspruch 3, dadurch gekennzeichnet, daß die Verbindungsleiste (40) mit ihren, jeweils einen Längsschlitz (50) aufweisenden Leistenendstücken mit jeweils einer Spannfläche der einander zugeordneten Orthesenteilhälften (10a, 10b bzw. 12a, 12b) mittels die Leistenendstücke durchsetzenden und in die Spannfläche einschraubbaren Spannschrauben (48) verspannbar ist.

5. Knieorthese nach Anspruch 4, dadurch gekennzeichnet, daß die Endstücke der Verbindungsleiste (40) mit jeweils einer taschenartigen Ausnehmung (42 bzw. 44) der Orthesenteilhälften (10a, 10b, 12a, 12b) in Eingriff sind.

6. Knieorthese nach einem der vorhergehenden Ansprüche, dadurch gekennzeichnet, daß die beiden Orthesenteile (10, 12) jeweils durch zwei in Beinlängsrichtung im Abstand voneinander angeordnete und miteinander verbundene, halbschalenförmige Teilstücke (10', 10'' bzw. 12', 12'') gebildet sind, deren gegenseitiger Abstand stufenlos verstellbar ist.

7. Knieorthese nach Anspruch 6, dadurch gekennzeichnet, daS die auf der vom Knie abgekehrten Seite vorhandenen Teilstücke (10', 12') beider Orthesenteile (10, 12) einstückig ausgebildet sind.

8. Knieorthese nach Anspruch 6 oder 7, dadurch gekennzeichnet, daß die einander zugeordneten Teilstücke (10', 10'' bzw. 12', 12'') beider Orthesenteile (10, 12) über eine weitere, sich in Beinlängsrichtung erstreckende Verbindungsleiste (46) miteinander verbunden sind.

9. Knieorthese nach Anspruch 8, dadurch gekennzeichnet, daß beide, jedem Orthesenteil (10, 12) zugeordneten Verbindungselemente (36, 38) durch ein T-Stück gebildet sind.

10. Knieorthese nach einem der vorhergehenden Ansprüche, dadurch gekennzeichnet, daß die Orthesenteile (10, 12) bzw. deren Teilstücke (10', 10'' bzw. 12', 12'') über jeweils mindestens zwei miteinander fest verbindbare Spannbänder (54) am Ober- bzw. Unterschenkel festlegbar sind, die an an den Orthesenteilen (10, 12) bzw. deren Teilstücken (10', 10'' bzw. 12', 12'') verschwenkbar befestigten Halteösen (56) gehalten sind.

11. Knieorthese nach einem der vorhergehenden Ansprüche, dadurch gekennzeichnet, daß beide Orthesenteile (10, 12) gegen die Wirkung einer Rückstellkraft aus der Orthesenstrecklage verschwenkbar sind.

12. Knieorthese nach Anspruch 11, dadurch gekennzeichnet, daß beide Orthesenteile (10, 12) über wenigstens einen an diesen befestigten und seitlich der Knieorthese verlaufenden und bei Beugen derselben spannbaren Kniestreckzug miteinander verbunden sind.

## Claims

1. Knee orthesis having substantially two orthesis parts (10, 12) in the form of half-shells, that can be applied directly to the thigh and the lower leg (32 and 34, respectively), and that are pivoted one on the other in one plane via lateral joints (16, 18),
**characterised in that**
at least certain sections (10'' and/or 12'') of the orthesis parts (10, 12) are divided along their apex (28, 30) and that the two halves of the orthesis parts (10a, 10b and 12a, 12b, respectively) can be adjusted and fixed in position relative one to the other, transversely to the longitudinal direction of the leg.

2. Knee orthesis according to claim 1, characterised in that said two halves (10a, 10b and 12a, 12b) of said orthesis parts (10, 12) can be adjusted relative to each other infinitely and independently.

3. Knee orthesis according to claim 1 or 2, characterised in that said two halves (10a, 10b, 12a, 12b) of said orthesis parts (10, 12) are interconnected by and guided on a connection strip (40), extending transversely to the leg, on which they can be adjusted and fixed.

4. Knee orthesis according to claim 3, characterised in that each of the end portions of said connection strip (40), being each provided with an elongated slot (50), is tightened against a tensioning surface on each of the associated halves of said orthesis parts (10a, 10b and 12a, 12b), by means of tensioning screws (48) that can be passed through the end portions of said strips and screwed into the tensioning surface.

5. Knee orthesis according to claim 4, characterised in that each of said end portions of said connection strip (40) is in engagement with a pocket-like recess (42 and 44, respectively) in the halves of said orthesis parts (10a, 10b, 12a, 12b).

6. Knee orthesis according to any of the preceding claims, characterised in that each of said two orthesis parts (10, 12) is formed by two interconnected sections (10', 10'' and 12', 12'', respectively) that are provided in spaced arrangement in the longitudinal direction of the leg and the mutual spacing of which can be infinitely adjusted.

7. Knee orthesis according to claim 6, characterised in that said sections (10', 12'), on the side facing away from the knee, of both orthesis parts (10, 12) are formed as a single piece.

8. Knee orthesis according to claim 6 or 7, characterised in that the respective associated sections (10', 10'' and 12', 12'', respectively) of both orthesis parts (10, 12) are interconnected via a further connection strip (46) extending in the longitudinal direction of the leg.

9. Knee orthesis according to claim 8, characterised in that the two connection elements (36, 38) associated with each orthesis part (10, 12) are formed by a T-shaped piece.

10. Knee orthesis according to any of the preceding claims, characterised in that said orthesis parts (10, 12) or their sections (10', 10'' and 12', 12'', respectively) can be fixed on the thigh and the lower leg, respectively, by at least two respective tensioning straps (54) that can be firmly connected one with the other and that are held in lugs (56) mounted pivotally on said orthesis parts (10, 12) or their sections (10', 10'' and 12', 12'', respectively).

11. Knee orthesis according to any of the preceding claims, characterised in that said two orthesis parts (10, 12) can be pivoted from the stretched position of the orthesis against the action of a restoring force.

12. Knee orthesis according to claim 11, characterised in that said two orthesis parts (10, 12) are interconnected by at least one knee-stretching train, which is fastened on said parts, extends laterally along the knee orthesis and can be tensioned as the knee bends.

## Revendications

1. Orthèse pour le genou avec respectivement deux parties d'orthèse (10, 12) semi-monocoques applicables directement sur la cuisse et la jambe (32 et 34) et reliées de façon pivotable entre elles sur un niveau au moyen d'articulations latérales (16, 18),
**caractérisée en ce que**
au moins des pièces partielles (10'' et 12'') des parties d'orthèse (10, 12) sont divisées le long de leur sommet (28 et 30) et les deux moitiés des parties d'orthèse (10a, 10b, 12a, 12b) sont pivotables et blocables l'une par rapport à l'autre perpendiculairement à la direction longitudinale de la jambe.

2. Orthèse pour le genou selon la revendication 1, caractérisée en ce que les deux moitiés (10a, 10b et 12a, 12b) des parties d'orthèse (10, 12) sont réglables en continu indépendamment l'une de l'autre et l'une par rapport à l'autre.

3. Orthèse pour le genou selon la revendication 1 ou 2, caractérisée en ce que les deux moitiés (10a, 10b et 12a, 12b) des parties d'orthèse (10, 12) sont reliées ensemble au moyen d'une tige de liaison (40) s'étendant perpendiculairement à la jambe et sont guidées sur cette tige de façon réglable et blocable.

4. Orthèse pour le genou selon la revendication 3, caractérisée en ce que la tige de liaison (40) peut être tendue avec ses pièces d'extrémité présentant respectivement une fente longitudinale (50) et avec respectivement une surface de serrage des moitiés partielles de l'orthèse (10a, 10b et 12a, 12b) conjuguées au moyen des vis de serrage (48) traversant les pièces d'extrémité de la tige et pouvant être vissés dans la surface de serrage.

5. Orthèse pour le genou selon la revendication 4, caractérisée en ce que les pièces d'extrémité de la tige de liaison (40) engrènent respectivement dans un évidement (42 et 44) en forme de poche des moitiés partielles de l'orthèse (10a, 10b, 12 a, 12b).

6. Orthèse pour le genou selon l'une des revendications précédentes, caractérisée en ce que les deux parties de l'orthèse (10, 12) sont formées respectivement par deux pièces partielles (10', 10'' et 12', 12'') semi-monocoques disposées en direction longitudinale de la jambe à un certain écart l'une de l'autre et reliées ensemble et dont l'écart réciproque est réglable.

7. Orthèse pour le genou selon la revendication 6, caractérisée en ce que les pièces partielles (10', 12') disponibles sur le côté opposé du genou des deux parties de l'orthèse (10, 12) sont confectionnées d'une pièce.

8. Orthèse pour le genou selon la revendication 6 ou 7, caractérisée en ce que les pièces partielles conjuguées (10', 10'' et 12', 12'') des deux parties de l'orthèse (10, 12) sont reliées ensemble par le biais d'une autre tige de liaison (46) s'étendant en direction longitudinale de la jambe.

9. Orthèse pour le genou selon la revendication 8, caractérisée en ce que les deux éléments de liaison (36, 38) conjugués à chaque partie (10, 12) de l'orthèse sont formés en pièce T.

10. Orthèse pour le genou selon l'une des revendications précédentes, caractérisée en ce que les parties (10, 12) de l'orthèse ou leurs pièces partielles (10', 10'' et 12', 12'') peuvent être fixées à la cuisse et à la jambe au moyen de respectivement au moins deux bandes de serrage (54) pouvant être reliées fermement ensemble et maintenues sur les parties d'orthèse (10, 12) et leurs pièces partielles (10', 10'' et 12', 12'') par le biais d'oeillets de retenu (56) pivotables.

11. Orthèse pour le genou selon l'une des revendications précédentes, caractérisée en ce que les deux parties de l'orthèse (10, 12) peuvent être pivotées de la position d'extension en antagonisme à une force de rappel.

12. Orthèse pour le genou selon la revendication 11, caractérisée en ce que les deux parties (10, 12) de l'orthèse sont reliées ensemble par au moins un train d'extension du genou fixé sur les deux parties et s'étendant latéralement de l'orthèse et pouvant être tendu en pliant l'orthèse.
